# EUROPÄISCHE PATENTSCHRIFT

(11) **EP 3 096 676 B1**
(45) Veröffentlichungstag und Bekanntmachung des Hinweises auf die Patenterteilung: **24.04.2019**
(21) Anmeldenummer: 15701748.4
(22) Anmeldetag: 22.01.2015
(51) Int. Cl.: A61B 1/267, G10L 25/90

(54) **NACHFÜHRUNG DER GRUNDFREQUENZ EINES STIMMSIGNALS IN ECHTZEIT**
TRACKING THE FUNDAMENTAL FREQUENCY OF A VOICE SIGNAL IN REAL TIME
SUIVI EN TEMPS RÉEL DE LA FRÉQUENCE DE BASE D'UN SIGNAL VOCAL

(30) Priorität: 24.01.2014 DE 102014201286
(43) Veröffentlichungstag der Anmeldung: 30.11.2016
(73) Patentinhaber: Digital Endoscopy GmbH, 86316 Friedberg (DE)
(72) Erfinder: PAUKER, Friedrich, 86420 Diedorf (DE)
(74) Vertreter: TBK
(86) Internationale Anmeldenummer: PCT/EP2015/051245
(87) Internationale Veröffentlichungsnummer: WO 2015/110525

(56) Entgegenhaltungen:
- WO-A1-00/33727
- WO-A1-2009/008596
- JP-A- 2002 291 699
- JP-A- 2002 291 699
- US-A- 3 549 806
- C. MANFREDI ET AL: "A comparative analysis of fundamental frequency estimation methods with application to pathological voices", MEDICAL ENGINEERING & PHYSICS, Bd. 22, Nr. 2, 1. März 2000 (2000-03-01), Seiten 135-147, XP055178612, ISSN: 1350-4533, DOI: 10.1016/S1350-4533(00)00018-7

## Beschreibung

Vorliegende Erfindung bezieht sich auf eine Nachführung eines Stimmsignals in Echtzeit.

Eine Grundfrequenzanalyse des Stimmsignals eines Probanden ist beispielsweise auf dem Gebiet der Stroboskopie erforderlich. Mittels der Stroboskopie kann die Beweglichkeit der Stimmlippen beurteilt werden. Die Triggerung einer Bildaufnahme der Stimmlippen erfolgt beispielsweise durch die Phonation des Probanden über ein Körper- oder Luftleitungsmikrofon.

Zum Initiieren der Phonation werden die Stimmlippen in Phonationsstellung gebracht, das heißt, sie liegen locker aneinander an und verschließen so die Glottis. Durch den Luftstrom werden die Stimmlippen in Schwingung versetzt, so dass die Luft mit jeder Öffnung und Schließung stoßweise in den Artikulationsraum entlassen wird. Es entstehen komplizierte periodische, aus Teiltönen bestehende Schwingungen. Die Frequenz, e.g. 70-1000 Hz, hängt dabei von der Länge der Stimmlippen ab.

Auf dem Gebiet der Stroboskopie wird ein Stroboskop beruhend auf dieser (Grund-)Frequenz angesteuert. Das an einem Endoskop angebrachte Stroboskop dient zur Aufnahme von Bildern der Stimmlippen zu geeigneten Zeitpunkten, die mittels der Grundfrequenz des Stimmsignals bestimmt werden.

Eine Aufgabe der Erfindung besteht in einer Vereinfachung eines Aufbaus zur Nachführung der Grundfrequenz eines Stimmsignals beispielsweise bei der Spektroskopie.

Diese Aufgabe wird durch das Verfahren und die Vorrichtung gemäß den beigefügten Patentansprüchen gelöst.

Erfindungsgemäß tastet eine Abtasteinrichtung ein Stimmsignal eines Probanden über einen vorbestimmten Zeitabschnitt ab, wodurch Abtastdaten des Stimmsignals erhalten werden, bildet ein Datenpaket aus den Abtastdaten, und sendet das Datenpaket zu einer Berechnungseinrichtung. Die Berechnungseinrichtung empfängt das Datenpaket und berechnet eine Grundfrequenz des Stimmsignals anhand der in dem Datenpaket enthaltenen Abtastdaten. Die Abtasteinrichtung setzt einen Zeitgeber beruhend auf der von der Berechnungseinrichtung berechneten Grundfrequenz und gibt ein Triggersignals bei Ablauf des Zeitgebers aus. Ein Vorteil der Erfindung liegt darin, dass Aufnahme des Stimmsignals und Analyse des Stimmsignals an verschiedenen Orten durchgeführt werden können, wodurch der Aufbau einer Aufnahmeeinrichtung verkleinert werden kann. Zudem ist es möglich, die Grundfrequenz des Stimmsignals asynchron zur Ansteuerung eines Stroboskops zu ermitteln, wodurch ein aufwendiger Aufbau zur Ermittlung der Grundfrequenz in Echtzeit vermieden werden kann.

### Stand der Technik:

Dokument WO 2009/008596 A1 (UMEDICAL CO LTD) offenbart ein Endoskop mit Stroboskop mit Mikrophon. Das Audiosignal wird aufgenommen und die Grundfrequenz ermittelt, womit ein Triggersignal erstellt wird.

Dokument JP 2002 291699 offenbart ebenfalls ein Endoskop mit Mikrophon und Kamera für eine Grundfrequenz-basierende Glottis Analyse. Das Mikrophon ist direkt am Endoskop angebracht.

Die Erfindung wird nachstehend anhand von Ausführungsbeispielen unter Bezugnahme auf die beiliegenden Zeichnungen näher beschreiben. Es zeigen:
Fig. 1 Ablaufdiagramme für einen Prozess 1, einen Prozess 2 und einen Prozess 3 zur Nachführung der Grundfrequenz eines Stimmsignals gemäß einem Ausführungsbeispiel der Erfindung.
Fig. 2 ein schematisches Blockschaltbild einer Vorrichtung zur Nachführung der Grundfrequenz eines Stimmsignals gemäß einem Ausführungsbeispiel der Erfindung.
Fig. 3 eine schematische Darstellung einer Nachführung der Grundfrequenz eines Stimmsignals gemäß einem Ausführungsbeispiel der Erfindung.

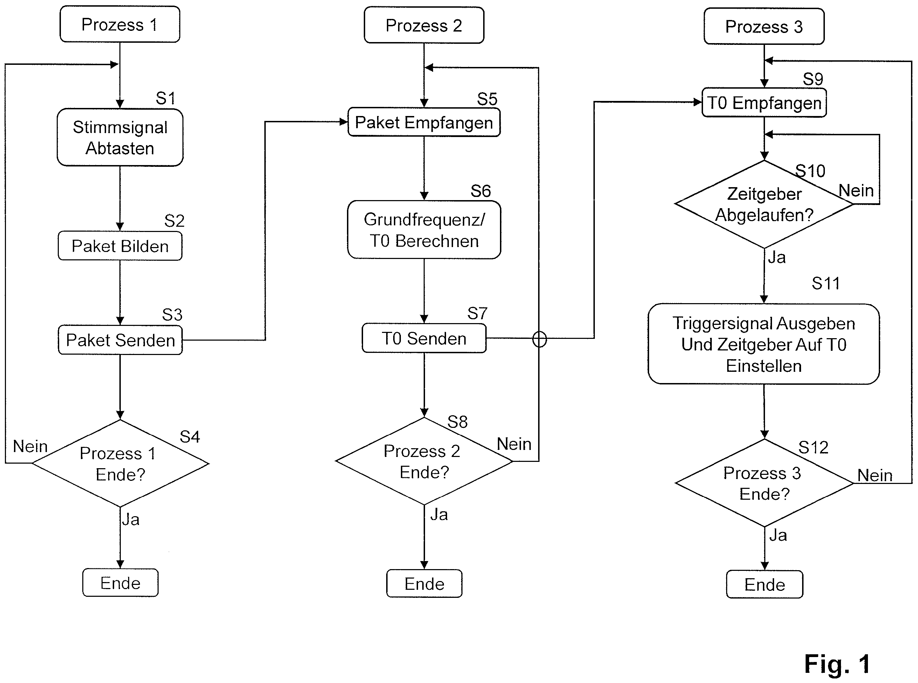

Fig. 1 veranschaulicht drei Prozesse, Prozess 1, Prozess 2 und Prozess 3, wobei die Prozesse 1 und 2 bzw. 3 und 2 jeweils an verschiedenen Orten implementiert sein können, und die Prozesse 1 und 3 an demselben Ort implementiert sein können.

Der Prozess 1 umfasst die Schritte: Abtasten (S1) eines Stimmsignals, beispielsweise eines Phonationsstroms, eines Probanden über einen vorbestimmten Zeitabschnitt, wodurch Abtastdaten des Stimmsignals erhalten werden, Bilden (S2) eines Datenpakets aus den Abtastdaten, Senden (S3) des Datenpakets zu einer Berechnungseinrichtung und Wiederholen dieser Schritte, solange Prozess 1 nicht beendet wird (Nein in S4).

Der Prozess 2 umfasst die Schritte: Empfangen (S5) des Datenpakets von einer Abtasteinrichtung, Berechnen (S6) einer Grundfrequenz f0 des Stimmsignals anhand der in dem Datenpaket enthaltenen Abtastdaten an der Berechnungseinrichtung und Berechnen von T0=1/f0, Senden (S7) von T0 zu der Abtasteinrichtung, und Wiederholen dieser Schritte, solange Prozess 2 nicht beendet wird (Nein in S8).

Der Prozess 3 umfasst die Schritte: Empfangen (S9) von T0 an der Abtasteinrichtung, Überprüfen (S10), ob ein Zeitgeber abgelaufen ist, Ausgeben eines Triggersignals und Setzen (S11) des Zeitgebers auf TO, wenn der Zeitgeber abgelaufen ist (Ja in S10), und Wiederholen dieser Schritte, solange Prozess 3 nicht beendet wird (Nein in S11).

Zu Beginn von Prozess 3, wenn noch kein T0 von Prozess 2 empfangen wurde, kann der Zeitgeber auf einen vorbestimmten Wert von T0 eingestellt werden. Ist der empfangene Wert von T0 von dem vorbestimmten Wert von T0 oder von dem zuvor eingestellten Wert von T0 verschieden, wird der Zeitgeber nach seinem Ablauf auf den empfangenen Wert von T0 eingestellt. Das heißt, der Zeitgeber wird immer auf den aktuellen Wert von T0 gesetzt.

Der Zeitgeber befindet sich vorzugsweise auf der Seite der Abtastvorrichtung, die die Abtastdaten sammelt. Bei Ablauf des Zeitgebers wird das Triggersignal zu einer Blitzlampe bzw. einem Stroboskop ausgegeben, um ein Blitzsignal beispielsweise für die Aufnahme der Stimmlippen durch eine Kamera zu erzeugen.

Um Sprünge zwischen einem zuvor in dem Zeitgeber einstellten Wert von T0 und einem neu in dem Zeitgeber einzustellenden Wert von T0 zu vermeiden, wird gemäß einem Ausführungsbeispiel der Erfindung der Übergang zwischen dem alten und neuen Wert von T0 gleitend, beispielsweise unter Verwendung eines bekannten Reglers durchgeführt.

Das bei Ablauf des Zeitgebers ausgegebene Triggersignal kann ein Stroboskop ansteuern, das beispielsweise nahe der Glottis des Probanden angeordnet ist und zusammen mit einer Kamera zur Aufnahme eines Bildes der Glottis dient.

In Schritt S1 wird das Stimmsignal beispielsweise mit einem Mikrophon aufgenommen, das nahe der Glottis des Probanden angeordnet ist.

Das in Schritt S2 gebildete Datenpaket enthält eine bestimmte Anzahl an Abtastwerten des Stimmsignals, die durch das Abtasten des Stimmsignals über den vorbestimmten Zeitabschnitt erfasst werden. Beispielsweise kann der Übergang von Schritt S1 zu Schritt S2 getriggert werden, wenn die bestimmte Anzahl der Abtastwerte erfasst ist. D.h., die Bildung des Datenpakets wird getriggert, wenn die bestimmte Anzahl der Abtastwerte erfasst ist.

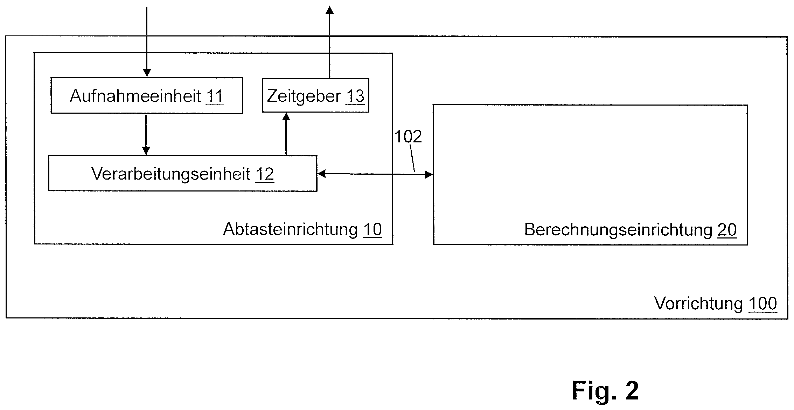

Fig. 2 zeigt ein schematisches Blockschaltbild einer Vorrichtung 100 zur Nachführung der Grundfrequenz eines Stimmsignals gemäß einem Ausführungsbeispiel der Erfindung.

Die Vorrichtung 100 umfasst eine Abtasteinrichtung 10 und eine Berechnungseinrichtung 20. Die vorstehend beschriebenen Prozesse 1 und 3 können in der Abtasteinrichtung 10 implementiert sein, die beispielsweise an einem Endoskop angebracht ist. Der Prozess 2 kann in der Berechnungseinrichtung 20 implementiert sein, die separat vorgesehen ist.

Die Abtasteinrichtung 10 umfasst eine Aufnahmeeinheit 11, eine Verarbeitungseinheit 12 und einen Zeitgeber 13. Die Aufnahmeeinheit 11 erhält das von dem Mikrophon aufgenommene Stimmsignal. Das Mikrophon kann durch die Aufnahmeeinheit 11 umfasst sein.

Die Verarbeitungseinheit 12 erhält das aufgenommene Stimmsignal von der Aufnahmeeinheit 11 und führt eine Abtastung des Stimmsignals mit einer bestimmten Abtastfrequenz durch. Dabei werden Abtastwerte erhalten, die eine bestimme Auflösung in Bits haben. Die Verarbeitungseinheit 12 fügt die Abtastwerte zu Datenpaketen mit einer bestimmten Bitlänge zusammen. D.h., ein Datenpaket enthält eine bestimmte Anzahl an Datenwerten. Wurde eine bestimmte Anzahl an Datenwerten durch die Abtastung erhalten, erzeugt die Verarbeitungseinheit 12 das Datenpaket und sendet es zu der Berechnungseinrichtung 20. Die Funktionen der Verarbeitungseinheit 12 können durch einen DSP (Digital Signal Processor), ein FPGA (Field Programmable Gate Array) oder dergleichen implementiert sein. Die Verarbeitungseinrichtung 12 kann auch einen Prozessor und einen Speicher umfassen, der ein Programm speichert, das bei Ausführung durch den Prozessor die vorstehend beschriebenen Funktionen der Verarbeitungseinrichtung 12 verwirklicht.

Die Datenpakete können über eine serielle Schnittstelle 102 zu der Berechnungseinrichtung 20 gesendet werden. Die Verbindung zwischen Abtasteinrichtung 10 und Berechnungseinrichtung 20 kann drahtgebunden oder drahtlos sein.

Die Berechnungseinrichtung 20 empfängt das Datenpaket von der Abtasteinrichtung 10 und berechnet anhand der darin enthaltenen Abtastwerte die Grundfrequenz f0 des Stimmsignals. Das heißt, die Berechnungseinrichtung 20 führt eine Frequenzanalyse des Stimmsignals anhand der in dem Datenpaket enthaltenen Abtastwerte durch. Die Berechnungseinrichtung 20 berechnet auch eine Zeit T0=1/f0. Die Berechnungseinrichtung 20 sendet T0 beispielsweise über die serielle Schnittstelle 102 zu der Abtasteinrichtung 10.

Die Funktionen der Berechnungseinrichtung 20 können durch einen DSP (Digital Signal Processor), ein FPGA (Field Programmable Gate Array) oder dergleichen implementiert sein. Die Berechnungseinrichtung 20 kann auch einen Prozessor und einen Speicher umfassen, der ein Programm speichert, das bei Ausführung durch den Prozessor die vorstehend beschriebenen Funktionen der Berechnungseinrichtung 20 verwirklicht.

Die von der Berechnungseinrichtung 20 gesendete Zeit T0 wird dem Zeitgeber 13 beispielsweise über die Verarbeitungseinheit 12 zugeführt. Der Zeitgeber 13 wird auf T0 gesetzt, und bei Ablauf der Zeit T0 wird ein Triggersignal beispielsweise zur Ansteuerung einer Blitzlampe bzw. eines Stroboskops ausgegeben.

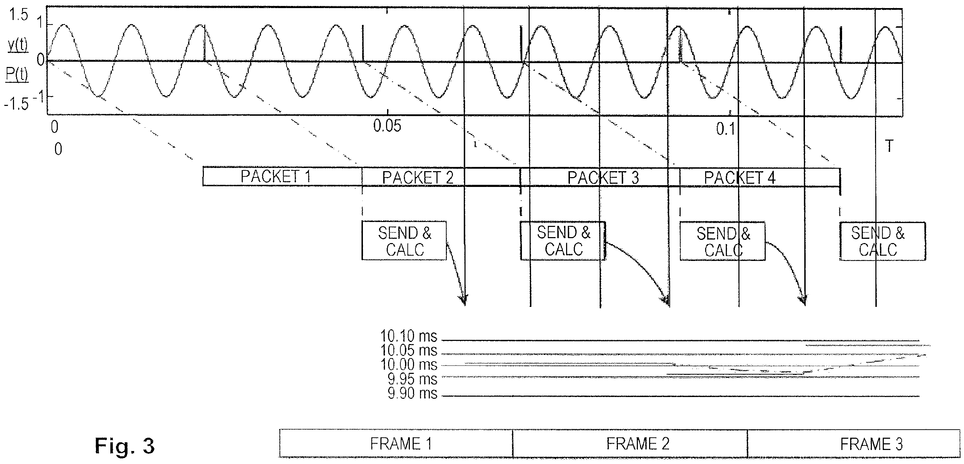

Fig. 3 zeigt eine Darstellung, die Erfassung und Nachführung einer Grundfrequenz f0 eines Stimmsignals gemäß einem Ausführungsbeispiel der Erfindung veranschaulicht.

y(t) stellt einen Signalverlauf eines Stimmsignals eines Probanden dar. Fig. 3 zeigt den Signalverlauf als Sinuswelle mit einer Frequenz von 100Hz. Beispielsweise kann die Aufnahmeeinheit 11 den Signalverlauf y(t) für die Verarbeitungseinheit 12 bereitstellen.

Die Abtastrate, mit der der Signalverlauf abgetastet wird, beträgt 44,1kHz, was einer Zeitdauer von ca. 22,7µs entspricht. Die Auflösung eines Abtastwerts beträgt 16Bit, und die Paketlänge umfasst 1023 Byte, was einer Zeitdauer von ca. 23,22ms entspricht. Es wird angemerkt, dass die Erfindung nicht auf diese Werte beschränkt ist. Ein Datenpaket kann beispielsweise zehn Schwingungen des Stimmsignals enthalten.

Wurden 1024 Byte an Abtastwerten erhalten, triggert ein Triggersignal P(t) das Erzeugen bzw. Fertigstellen und Senden des Datenpakets. Fig. 3 zeigt Datenpakete Packet 1, Packet 2, Packet 3 und Packet 4, deren Erzeugung und Senden durch P(t) getriggert werden.

Das vorstehend beschriebene Abtasten des Signalverlaufs y(t) und die Erzeugung und das Senden der Datenpakete können im in Fig. 1 gezeigten Prozess 1 bzw. durch die Abtasteinrichtung 10 in Fig. 2 durchgeführt werden.

Anhand der in den Datenpaketen enthaltenen Abtastwerte wird die Grundfrequenz f0 des Signalverlaufs y(t) berechnet. f0 wird erstmals anhand des Datenpakets Packet 1 berechnet. Fig. 3 veranschaulicht die Verzögerung zwischen Signalverlaufabtastung, Paketerzeugung, Senden des Pakets, Berechnung von f0 und T0 und Setzen des Zeitgebers auf T0.

Mit der erstmaligen Berechnung von f0 wird der Zeitgeber auf T0=1/f0 gesetzt. Hat sich nach Verarbeitung des Datenpakets Packet 2 f0 verändert, wird der Zeitgeber nach Ablauf der zuvor gesetzten Zeit auf die neu berechnete Zeit eingestellt, oder der alte Wert von T0 und der neue Wert von T0 können gemittelt werden. Es ist auch möglich, eine konstante Phase oder eine mit der Zeit ansteigende Phase zu T0 zu addieren, um einen gleitenden Übergang zwischen altem und neuem Wert von T0 zu ermöglichen.

Mit der vorstehend beschriebenen Konfiguration kann die Grundfrequenz f0 über die Zeit nachgeführt werden. Insbesondere wird beruhend auf dem zum Stimmsignal asynchronen Vorgang des Verarbeitens der Datenpakete wieder ein zum Stimmsignal synchrones Echtzeit-Triggersignal für die Blitzlampe bzw. das Stroboskop erzeugt.

Beispielsweise ist ein Stroboskop an einem Endoskop angebracht, das sich in der Nähe der Glottis des Probanden befindet. Anhand des Triggersignals erzeugt das Stroboskop einen Lichtblitz, und eine am Endoskop angebrachte Kamera nimmt ein Bild der Stimmlippen auf. Fig. 3 veranschaulicht die aufgenommenen Bilder als asynchrone Bilddatenpakete Frame 1, Frame 2, Frame 3.

Die vorstehend beschriebene Berechnung der Grundfrequenz f0 kann im in Fig. 1 gezeigten Prozess 2 bzw. durch die Berechnungseinrichtung 20 in Fig. 2 durchgeführt werden. Die Erzeugung des Triggersignals kann durch den Prozess 3 bzw. die Abtasteinrichtung 10, insbesondere durch Setzen des Zeitgebers 13 durchgeführt werden.

Die Verzögerung zwischen erster Abtastung für ein Datenpaket und Ansteuerung mit (neu) berechneter Grundfrequenz f0 beträgt im in Fig. 3 gezeigten Beispiel ca. 61,64ms, was aber für die Aufnahme geeigneter Bilder der Stimmlippen vernachlässigbar ist.

Erfindungsgemäß werden durch Abtastung eines Stimmsignals erhaltene Abtastdaten in Datenpaketen zu einer Einrichtung gesendet, wo sie zum Erfassen der Grundfrequenz des Stimmsignals verarbeitet werden. Dadurch kann der Aufbau an der Abtasteinrichtung verkleinert werden. Zudem ist es möglich, die Grundfrequenz des Stimmsignals asynchron zur Ansteuerung eines Stroboskops zu ermitteln, wodurch ein aufwendiger Aufbau zur Ermittlung der Grundfrequenz in Echtzeit vermieden werden kann.

## Patentansprüche

1. Verfahren für eine Vorrichtung (100) zur Nachführung einer Grundfrequenz eines Stimmsignals, wobei die Vorrichtung (100) eine an einem Endoskop angebrachte Abtasteinrichtung (10), eine Berechnungseinrichtung (20) und eine serielle Schnittstelle (102) enthält, die die Berechnungseinrichtung (20) und die Abtasteinrichtung (10) verbindet, wobei die Abtasteinrichtung (10) ein Mikrophon (11) umfasst, das nahe der Glottis eines Probanden angeordnet ist und ein Stimmsignal des Probanden aufnimmt, wobei das Verfahren die Schritte umfasst:
(a) Abtasten (S1) des Stimmsignals des Probanden über einen vorbestimmten Zeitabschnitt an der Abtasteinrichtung, wodurch Abtastdaten des Stimmsignals erhalten werden, wobei der vorbestimmte Zeitabschnitt eine bestimmte Anzahl an Schwingungen des Stimmsignals umfasst, und Triggern von Schritt (b), wenn eine bestimmte Anzahl an Abtastwerten durch das Abtasten des Stimmsignals über den vorbestimmten Zeitabschnitt erfasst ist,
(b) Bilden (S2) eines Datenpakets aus den Abtastdaten an der Abtasteinrichtung, wobei das Datenpaket die bestimmte Anzahl an Abtastwerten des Stimmsignals enthält,
(c) Senden (S3) des Datenpakets von der Abtasteinrichtung über die serielle Schnittstelle (102) zu der Berechnungseinrichtung,
(d) Berechnen (S6) einer Grundfrequenz des Stimmsignals anhand der in dem Datenpaket enthaltenen Abtastdaten an der Berechnungseinrichtung und Senden der berechneten Grundfrequenz über die serielle Schnittstelle zu der Abtasteinrichtung,
(e) Setzen eines Zeitgebers beruhend auf der von der Berechnungseinrichtung gesendeten Grundfrequenz, und
(f) Ausgeben eines Triggersignals bei Ablauf des Zeitgebers,
wobei die Schritte (a) bis (d) eine bestimmte Anzahl oft wiederholt werden, Schritt (e) nach Ablauf des Zeitgebers durchgeführt wird, und Schritt (d) asynchron zu den Schritten (a) bis (c), (e) und (f) durchgeführt wird, und
wobei die Schritte (a) bis (c), (e) und (f) durch einen Prozessor der Abtasteinrichtung (10) durchgeführt werden, der ein in einem Speicher der Abtasteinrichtung (10) gespeichertes Programm ausführt, und
wobei Schritt (d) durch einen Prozessor der Berechnungseinrichtung (20) durchgeführt wird, der ein in einem Speicher der Berechnungseinrichtung (20) gespeichertes Programm ausführt.

2. Verfahren nach Anspruch 1, ferner mit dem Schritt:
Ausgeben des Triggersignals zu einem an dem Endoskop angebrachten Stroboskop, das nahe der Glottis des Probanden angeordnet ist.

3. Verfahren nach Anspruch 1, wobei das Setzen des Zeitgebers beruhend auf der von der Berechnungseinrichtung gesendeten Grundfrequenz an der Abtasteinrichtung durchgeführt wird, und das Ausgeben eines Triggersignals bei Ablauf des Zeitgebers durch die Abtasteinrichtung durchgeführt wird.

4. Verfahren nach einem der Ansprüche 1 bis 3, wobei das Stimmsignal ein Phonationsstrom ist.

5. Vorrichtung (100) zur Nachführung einer Grundfrequenz eines Stimmsignals, mit:
einer Abtasteinrichtung (10), die an einem Endoskop angebracht ist, wobei die Abtasteinrichtung (10) ein Mikrophon (11) umfasst, das nahe der Glottis eines Probanden angeordnet werden kann und zur Aufnahme eines Stimmsignals des Probanden eingerichtet ist,
einer Berechnungseinrichtung (20), und
einer seriellen Schnittstelle (102), die die Berechnungseinrichtung (20) und die Abtasteinrichtung (10) verbindet,
wobei die Abtasteinrichtung zum (a) Abtasten des Stimmsignals des Probanden über einen vorbestimmten Zeitabschnitt, wodurch Abtastdaten des Stimmsignals erhalten werden, wobei der vorbestimmte Zeitabschnitt eine bestimmte Anzahl an Schwingungen des Stimmsignals umfasst, wenn eine bestimmte Anzahl an Abtastwerten durch das Abtasten des Stimmsignals über den vorbestimmten Zeitabschnitt erfasst ist, Triggern des Bildens eines Datenpakets aus den Abtastdaten und Senden des Datenpakets über die serielle Schnittstelle (102) zu der Berechnungseinrichtung (20) eingerichtet ist, wobei das Datenpaket die bestimmte Anzahl an Abtastwerten des Stimmsignals enthält,
wobei die Berechnungseinrichtung (20) zum Empfangen des Datenpakets über die serielle Schnittstelle (102), Berechnen einer Grundfrequenz des Stimmsignals anhand der in dem Datenpaket enthaltenen Abtastdaten und Senden der berechneten Grundfrequenz über die serielle Schnittstelle (102) zu der Abtasteinrichtung (10) eingerichtet ist,
wobei die Abtasteinrichtung (10) zum (b) Setzen eines Zeitgebers beruhend auf der von der Berechnungseinrichtung (20) gesendeten Grundfrequenz und Ausgeben eines Triggersignals bei Ablauf des Zeitgebers eingerichtet ist,
und wobei die Berechnungseinrichtung zum Berechnen der Grundfrequenz und Senden der berechneten Grundfrequenz über die serielle Schnittstelle (102) zu der Abtasteinrichtung asynchron zu den Verarbeitungsvorgängen (a) und (b) der Abtasteinrichtung eingerichtet ist, und
wobei die Abtasteinrichtung (10) einen Prozessor und einen Speicher umfasst, wobei der Prozessor der Abtasteinrichtung die Schritte (a) bis (c), (e) und (f) durch Ausführen eines in dem Speicher der Abtasteinrichtung gespeicherten Programms durchführt, und
wobei die Berechnungseinrichtung (20) einen Prozessor und einen Speicher umfasst, wobei der Prozessor der Berechnungseinrichtung Schritt (d) durch Ausführen eines in dem Speicher der Berechnungseinrichtung gespeicherten Programms durchführt.

6. Vorrichtung nach Anspruch 5, wobei die Abtasteinrichtung (10) zur Ausgabe des Triggersignals zu einem Stroboskop eingerichtet ist, das an dem Endoskop angebracht ist.

## Claims

1. Method for a device (100) for tracking a fundamental frequency of a voice signal, wherein the device (100) contains a sampling apparatus (10) attached to an endoscope, a calculation apparatus (20) and a serial interface (102) that connects the calculation apparatus (20) and the sampling apparatus (10), wherein the sampling apparatus (10) comprises a microphone (11) that is positioned close to the glottis of a test subject and records a voice signal of the test subject, wherein the method comprises the steps:
(a) sampling (S1) the voice signal of the test subject over a predetermined time interval on the sampling apparatus, by way of which sampling data of the voice signal are obtained, wherein the predetermined time interval comprises a particular number of oscillations of the voice signal, and triggering step (b) when a particular number of sample values have been acquired by sampling the voice signal over the predetermined time interval,
(b) forming (S2) a data packet from the sampling data on the sampling apparatus, wherein the data packet contains the particular number of sample values of the voice signal,
(c) transmitting (S3) the data packet from the sampling apparatus to the calculation apparatus via the serial interface (102),
(d) calculating (S6) a fundamental frequency of the voice signal using the sampling data contained in the data packet on the calculation apparatus, and transmitting the calculated fundamental frequency to the sampling apparatus via the serial interface,
(e) setting a timer based on the fundamental frequency transmitted by the calculation apparatus, and
(f) outputting a trigger signal when the timer expires,
wherein steps (a) to (d) are repeated a particular number of times, step (e) is performed after the timer expires, and step (d) is performed asynchronously with respect to steps (a) to (c), (e) and (f), and
wherein steps (a) to (c), (e) and (f) are performed by a processor of the sampling apparatus (10), which processor executes a program stored in a memory of the sampling apparatus (10), and
wherein step (d) is performed by a processor of the calculation apparatus (20), which processor executes a program stored in a memory of the calculation apparatus (20) .

2. Method according to Claim 1, furthermore having the step:
outputting the trigger signal to a stroboscope that is attached to the endoscope and is positioned close to the glottis of the test subject.

3. Method according to Claim 1, wherein the timer is set based on the fundamental frequency transmitted from the calculation apparatus to the sampling apparatus, and a trigger signal is output by the sampling apparatus when the timer expires.

4. Method according to one of Claims 1 to 3, wherein the voice signal is a phonation current.

5. Device (100) for tracking a fundamental frequency of a voice signal, having:
a sampling apparatus (10) that is attached to an endoscope, wherein the sampling apparatus (10) comprises a microphone (11) that is able to be positioned close to the glottis of a test subject and is configured so as to record a voice signal of the test subject,
a calculation apparatus (20), and
a serial interface (102) that connects the calculation apparatus (20) and the sampling apparatus (10),
wherein the sampling apparatus is configured so as to (a) sample the voice signal of the test subject over a predetermined time interval, by way of which sampling data of the voice signal are obtained, wherein the predetermined time interval comprises a particular number of oscillations of the voice signal, when a particular number of sample values have been acquired by sampling the voice signal over the predetermined time interval, trigger the formation of a data packet from the sampling data, and transmit the data packet to the calculation apparatus (20) via the serial interface (102), wherein the data packet contains the particular number of sample values of the voice signal,
wherein the calculation apparatus (20) is configured so as to receive the data packet via the serial interface (102), calculate a fundamental frequency of the voice signal using the sampling data contained in the data packet, and transmit the calculated fundamental frequency to the sampling apparatus (10) via the serial interface (102),
wherein the sampling apparatus (10) is configured so as to (b) set a timer based on the fundamental frequency transmitted by the calculation apparatus (20) and output a trigger signal when the timer expires,
and wherein the calculation apparatus is configured so as to calculate the fundamental frequency and transmit the calculated fundamental frequency to the sampling apparatus via the serial interface (102) asynchronously with respect to the processing procedures (a) and (b) of the sampling apparatus, and
wherein the sampling apparatus (10) comprises a processor and a memory, wherein the processor of the sampling apparatus performs steps (a) to (c), (e) and (f) by executing a program stored in the memory of the sampling apparatus, and
wherein the calculation apparatus (20) comprises a processor and a memory, wherein the processor of the calculation apparatus performs step (d) by executing a program stored in the memory of the calculation apparatus.

6. Device according to Claim 5, wherein the sampling apparatus (10) is configured so as to output the trigger signal to a stroboscope that is attached to the endoscope.

## Revendications

1. Procédé pour un arrangement (100) de poursuite d'une fréquence fondamentale d'un signal vocal, l'arrangement (100) contenant un dispositif d'échantillonnage (10) monté sur un endoscope, un dispositif de calcul (20) et une interface série (102) qui relie le dispositif de calcul (20) et le dispositif d'échantillonnage (10), le dispositif d'échantillonnage (10) comportant un microphone (11) qui est disposé à proximité de la glotte d'un sujet et enregistre un signal vocal du sujet, le procédé comprenant les étapes suivantes :
(a) échantillonnage (S1) du signal vocal du sujet sur un intervalle de temps prédéfini au niveau du dispositif d'échantillonnage, des données d'échantillonnage du signal vocal étant ainsi obtenues, l'intervalle de temps prédéfini comprenant un nombre défini d'oscillations du signal vocal, et déclenchement de l'étape (b) lorsqu'un nombre défini de valeurs échantillonnées est acquis par l'échantillonnage du signal vocal sur l'intervalle de temps prédéfini,
(b) formation (S2) d'un paquet de données à partir des données d'échantillonnage au niveau du dispositif d'échantillonnage, le paquet de données contenant le nombre défini de valeurs échantillonnées du signal vocal,
(c) envoi (S3) du paquet de données depuis le dispositif d'échantillonnage au dispositif de calcul par le biais de l'interface série (102),
(d) calcul (S6) d'une fréquence fondamentale du signal vocal à l'aide des données d'échantillonnage contenues dans le paquet de données au niveau du dispositif de calcul et envoi de la fréquence fondamentale calculée au dispositif d'échantillonnage par le biais de l'interface série,
(e) activation d'un minuteur en s'appuyant sur la fréquence fondamentale envoyée par le dispositif de calcul, et
(f) délivrance d'un signal de déclenchement à l'écoulement du minuteur,
les étapes (a) à (d) étant souvent répétées un nombre de fois défini, l'étape (e) étant mise en oeuvre après l'écoulement du minuteur et l'étape (d) étant mise en oeuvre de manière asynchrone par rapport aux étapes (a) à (c), (e) et (f), et
les étapes (a) à (c), (e) et (f) étant mises en oeuvre par un processeur du dispositif d'échantillonnage (10), lequel exécute un programme enregistré dans une mémoire du dispositif d'échantillonnage (10), et
l'étape (d) étant mise en oeuvre par un processeur du dispositif de calcul (20), lequel exécute un programme enregistré dans une mémoire du dispositif de calcul (20).

2. Procédé selon la revendication 1, comprenant en outre l'étape :
délivrance du signal de déclenchement à un stroboscope monté au niveau de l'endoscope, lequel est disposé à proximité de la glotte du sujet.

3. Procédé selon la revendication 1, l'activation du minuteur étant effectuée au niveau du dispositif d'échantillonnage en s'appuyant sur la fréquence fondamentale envoyée par le dispositif de calcul et la délivrance d'un signal de déclenchement par le dispositif d'échantillonnage étant effectuée à l'écoulement du minuteur.

4. Procédé selon l'une des revendications 1 à 3, le signal vocal étant un courant de phonation.

5. Arrangement (100) de poursuite d'une fréquence fondamentale d'un signal vocal, comprenant un dispositif d'échantillonnage (10), qui est monté sur un endoscope, le dispositif d'échantillonnage (10) comportant un microphone (11) qui peut être disposé à proximité de la glotte d'un sujet et qui est conçu pour enregistrer un signal vocal du sujet,
un dispositif de calcul (20), et
une interface série (102) qui relie le dispositif de calcul (20) et le dispositif d'échantillonnage (10),
le dispositif d'échantillonnage étant conçu pour (a) échantillonner le signal vocal du sujet sur un intervalle de temps prédéfini, des données d'échantillonnage du signal vocal étant ainsi obtenues, l'intervalle de temps prédéfini comprenant un nombre défini d'oscillations du signal vocal, lorsqu'un nombre défini de valeurs échantillonnées est acquis par l'échantillonnage du signal vocal sur l'intervalle de temps prédéfini, pour déclencher la formation d'un paquet de données à partir des données d'échantillonnage et pour envoyer le paquet de données au dispositif de calcul (20) par le biais de l'interface série (102), le paquet de données contenant le nombre défini de valeurs échantillonnées du signal vocal,
le dispositif de calcul (20) étant conçu pour recevoir le paquet de données par le biais de l'interface série (102), pour calculer une fréquence fondamentale du signal vocal à l'aide des données d'échantillonnage contenues dans le paquet de données et pour envoyer la fréquence fondamentale calculée au dispositif d'échantillonnage (10) par le biais de l'interface série (102),
le dispositif d'échantillonnage (10) étant conçu pour (b) activer un minuteur en s'appuyant sur la fréquence fondamentale envoyée par le dispositif de calcul (20) et pour délivrer un signal de déclenchement à l'écoulement du minuteur,
et le dispositif de calcul étant conçu pour calculer la fréquence fondamentale et pour envoyer la fréquence fondamentale calculée au dispositif d'échantillonnage par le biais de l'interface série (102) de manière asynchrone par rapport aux opérations de traitement (a) et (b) du dispositif d'échantillonnage, et
le dispositif d'échantillonnage (10) comprenant un processeur et une mémoire, le processeur du dispositif d'échantillonnage mettant en oeuvre les étapes (a) à (c), (e) et (f) en exécutant un programme enregistré dans la mémoire du dispositif d'échantillonnage, et
le dispositif de calcul (20) comprenant un processeur et une mémoire, le processeur du dispositif de calcul mettant en oeuvre l'étape (d) en exécutant un programme enregistré dans la mémoire du dispositif de calcul.

6. Arrangement selon la revendication 5, le dispositif d'échantillonnage (10) étant conçu pour délivrer le signal de déclenchement à un stroboscope qui est monté au niveau de l'endoscope.
